# EUROPEAN PATENT APPLICATION

(11) **EP 4 467 063 A1**
(43) Date of publication of application: **27.11.2024**
(21) Application number: 24177330.8
(22) Date of filing: 22.05.2024
(51) Int. Cl.: A61B 3/00, A61B 3/10, A61B 3/107, A61B 3/12, A61B 3/103, A61B 3/113

(54) **OPHTHALMIC IMAGING DEVICE**

(30) Priority: 24.05.2023 CH 5502023
(71) Applicant: Ziemer Ophthalmic Systems AG, 2562 Port (CH)
(72) Inventor: RATHJEN, Christian, 28197 Bremen (DE); STEINLECHNER, Michael, 8006 Zürich (CH)
(74) Representative: Rentsch Partner AG

(57) **Abstract**

Disclosed is an ophthalmic imaging device (1) comprising an on-axis measuring unit (2) and an off-axis iris imaging unit (3) the off-axis iris imaging unit (3) arranged at a predetermined angle (θ) to a measurement axis (M) of the ophthalmic imaging device (1) and comprising a sensor (31) defining an image plane, and an optical system (33) oriented relative to the image plane (32) such that a focal plane (34) of the off-axis iris imaging unit (3) substantially coincides with an iris plane (54) of the iris (51) of the eye (5).

## Description

### FIELD OF THE DISCLOSURE

The present invention relates to an ophthalmic imaging device.

### BACKGROUND OF THE DISCLOSURE

Ophthalmic measuring devices for the determination of the cornea and eye geometry, such as topography, measurement of eye length, and wavefront measurement systems, usually consist of several individual measuring units configured to measure the eye along the measurement axis of the measuring device, the measurement axis being substantially coincidental with an eye axis of the eye, wherein the eye axis is, or largely corresponds to, one of the following axes of the eye: an optical axis, a line of sight, and/or a visual axis. The eye axis may deviate from an optical axis of the eye by an angle. The ophthalmic measuring device achieves coaxiality (i.e., each of the measuring units measures the eye along the eye axis) through the use of beam splitters and/or aperture dividers which direct light received from the eye along the measurement axis to the individual measuring units, each individual measuring unit receiving a part of the direct light.

The following measurement units, for example, are arranged to image or measure the eye along the measurement axis and can include an infrared camera for recording a placido image and/or an optical coherence tomography (OCT) system. Further, lighting units may also be arranged along, or coincident with, the measurement axis: a fixation light, a split lamp for Scheimpflug illumination, or structured light, such as a laser or a Placido screen.

The fixation light is typically arranged on the measurement axis, such that, when the patient looks at the fixation light, the eye axis substantially coincides with the measurement axis of the measuring device.

EP2845534A1, US2015/335234A1, EP2932888A1, US2021/267799A1, US2021/093193A1, US2015/208916A1 and US2019/357767A1 are known prior art.

The known prior art, in particular the above-mentioned documents, disclose cameras which are arranged off-axis from the optical axis of the eye and configured to image the eye. However, all of the off-axis cameras disclosed in the aforementioned prior art are oriented directly towards the eye, in particular in that the sensor axis and optical system axis of the cameras are directly pointed at the eye. Thereby, the focal plane of the cameras does not coincide with an iris plane of the eye, but rather may only intersect the iris plane at an angle corresponding to the angle formed between the optical axis of the eye and the optical axis of the camera.

### SUMMARY OF THE DISCLOSURE

It is an object of embodiments disclosed herein to provide an ophthalmic iris imaging device. In particular, it is an object of the embodiments disclosed herein to provide an ophthalmic iris imaging device which does not have at least some of the drawbacks of the prior art.

The ophthalmic imaging device comprises an on-axis measuring unit configured to record one or more measurements of an eye. The on-axis measuring unit is configured to measure the eye along a measurement axis. The measurement axis is substantially coincidental to an eye axis of the eye. The ophthalmic imaging device comprises an off-axis iris imaging unit configured to record an off-axis iris image of an iris of the eye. The off-axis iris imaging unit is arranged at a predetermined angle to the measurement axis. The off-axis iris imaging unit comprises a sensor defining an image plane. The off-axis iris imaging unit comprises an optical system oriented relative to the image plane such that a focal plane of the off-axis iris imaging unit substantially coincides with an iris plane of the iris of the eye.

An advantage of the ophthalmic imaging device disclosed is that, due to arranging the off-axis iris imaging unit on a different axis than the on-axis measuring unit, less compromises must be made in the optical design of the on-axis measuring unit as no additional beam splitters are required to split off light towards an iris imaging unit configured for on-axis imaging of the iris of the eye. Not only do beam splitters reduce the amount of light available for downstream measuring units and/or imaging units, but these beam splitters often require specialized coatings and occupy further space in the ophthalmic imaging device. The ophthalmic imaging device disclosed therefore provides for a more compact on-axis measuring unit with less optical compromises.

The off-axis iris image preferably includes a color image, i.e. including wavelengths in the visible light spectrum of approx.. 380 nm to 700 nm. Additionally and/or alternatively, the off-axis iris image includes an infra-red image.

The off-axis iris imaging unit is, alternatively or additionally, arranged at a predetermined distance to the measurement axis.

The optical system may have an imaging axis. The imaging axis is oriented relative to the image plane of the sensor by a predefined angle such that the iris of the eye is in focus, in particular that at least a substantial proportion of the iris eye is in focus.

The off-axis iris imaging unit is preferably arranged at a distance of approximately 50-300 mm from the eye, the predetermined angle preferably being less than 45 degrees to the measurement axis (point of for defining the predetermined angle origin being the eye). The off-axis iris imaging unit is preferably arranged horizontally adjacent to the on-axis measuring unit. In other words, the off-axis iris imaging unit is preferably arranged next to the on-axis measuring unit. More specifically, the off-axis iris imaging unit is arranged on a horizontal plane coincident with the measurement axis and orthogonal to a vertical axis.

The off-axis iris imaging unit is preferably configured such that the iris is resolved with a lateral resolution of below 50 micrometers per pixel, preferably below 20 micrometers per pixel, most preferably below 15 micrometers per pixel. The off-axis iris imaging unit is preferably configured to generate an image having at least 1000 pixels by 1000 pixels. The off-axis iris imaging unit is preferably configured such that the depth of field is greater than 0.5 mm, preferably greater than 1 mm.

In an embodiment, the ophthalmic imaging device comprises a processing unit configured to correct the off-axis iris image. The processing unit is configured to correct the off-axis iris image by transforming the off-axis iris image to compensate for distortion. The distortion is caused by perspective, characteristics of the optical system, and/or characteristics of the eye.

The characteristics of the optical system may generate distortion (e.g., barrel distortion, pincushion distortion, trapeze distortion). The distortion may include radial distortion that depends on wavelength (also known as chromatic aberration).

The characteristics of the eye may include a curvature of the iris and optical properties of the cornea, in particular refractive properties of the cornea. The refractive properties of the cornea are due to the curvature of exterior corneal surface, material of cornea, thickness profile of cornea, curvature of interior corneal surface, and asphericity of cornea.

The transformation may be a geometric transformation or projection designed to correct for distortions and/or warpings.

In an embodiment, the processing unit is configured to use a digital eye model to correct for refractive properties of the cornea.

The off-axis iris image may be corrected using a transformation (e.g., affine transformation) and a standard/default digital eye model, which digital eye model is used to define parameters of the transformation. Additionally or alternatively, ray tracing through the digital eye model may be used to correct for distortion.

In an embodiment, the digital eye model is a parametrized digital eye model. The parametrized digital eye model may include parameters for characteristics of the eye. The characteristics of the eye include a diameter of the eye, a curvature of an anterior corneal surface, a curvature of a posterior corneal surface, a thickness profile of a cornea, an astigmatism of the cornea, and/or a curvature of the iris.

In an embodiment, the ophthalmic imaging device comprises a processing unit configured to determine at least one of the characteristics of the eye using the one or more measurements performed by the on-axis measuring unit.

In an embodiment, the ophthalmic imaging device comprises a processing unit configured to determine a tilt of the eye using the off-axis iris image. The tilt is relative to the measurement axis. The processing unit is configured to generate an untilted off-axis iris image by transforming the off-axis iris image based on the tilt of the eye.

The processor may be configured to determine the tilt of the eye by locating the center of the pupil in the off-axis iris image. The processor is configured to compare the location of the pupil to a location of one or more reflections, in the off-axis iris image, of one or more point light sources. The point light sources may include one or more point light sources which are part of the ophthalmic imaging device and/or include one or more point light sources which are external to the ophthalmic imaging device. The point light sources may reflect off the cornea of the eye, for example, forming bright spots in the off-axis iris image.

Alternatively, or additionally, the processor may be configured to determine the tilt of the eye by analyzing reflections of one or more structured light sources. The processor may be configured to determine the tilt of the eye by triangulation, using the analyzed reflections. The structured light sources may include one or more structured light sources which are part of the ophthalmic imaging device and/or include one or more structured light sources which are external to the ophthalmic imaging device. The structured light sources may be configured to project lines, curves, and/or patterns onto the eye. The reflections of the one or more structured light sources on the cornea may in particular be analyzed.

In an embodiment, the ophthalmic imaging device further comprises an eye tracker configured to measure a tilt of the eye, and a processing unit configured to receive, from the eye tracker, the measured tilt of the eye, and generate an untilted off-axis iris image by transforming the off-axis iris image based on the measured tilt of the eye.

In an embodiment, the ophthalmic imaging device is configured to simultaneously record at least one of the measurements of the eye and the off-axis iris image. Simultaneous recording means that the at least one measurement of the eye and the off-axis iris image are recorded at substantially the same time point.

Alternatively, the time-points of the measurement and the off-axis iris image can be recorded and compared by the ophthalmic imaging device.

In an embodiment, the ophthalmic imaging device comprises a processing unit configured to generate a personalized digital eye model of the eye of the patient, using the one or more measurements of the eye and the off-axis iris image.

The personalized digital eye model may be generated to include one or more 2D or 3D models of parts of the eye (e.g., including the iris and cornea).

The personalized digital eye model may be generated include a corneal topography map of the cornea of the eye. The corneal topography map is a topography of the cornea augmented, which may be augmented, by the processor, using the off-axis iris image (e.g. using an overlaid image of the iris). The corneal topography map may be converted, by the processor, into a 3D model of the cornea.

The personalized digital eye model may be generated to include tomography data.

The processing unit may be further configured to display, on a display, the personalized digital eye model.

In an embodiment, the on-axis measuring unit comprises an optical coherence tomography unit, an infrared placido imaging unit, an aberrometer, an optical biometer, and/or an auto-refractor.

The off-axis iris image may be used, by the processor, to generate a colored version of an on-axis iris image, for example a placido image. In particular, the off-axis iris image, by virtue of tilting and/or shifting the optical system with respect to the sensor, may appear substantially as an on-axis iris image, with or without subsequent correction.

The ophthalmic imaging device may further comprise a Scheimpflug imaging unit arranged off-axis to the measurement axis at an azimuthal angle with respect to the measurement axis different to an azimuthal angle of the off-axis iris imaging unit.

The Scheimpflug imaging unit may be used to generate tomographic images of the eye of the eye.

In an embodiment, the ophthalmic imaging device further comprises a further off-axis imaging unit. The further off-axis imaging unit is arranged at a predetermined angle to the measurement axis and at a predetermined azimuthal angle about the measurement axis with respect to the azimuthal angle of the off-axis imaging unit. The further off-axis imaging unit is configured to record a further off-axis iris image of the iris of the eye. A processing unit is configured to merge the off-axis iris image and the further off-axis iris image to generate a combined off-axis iris image. Thereby, for example, a shadowing of part of the iris (due to the limbus or other eye features such as eye lids) can be compensated for.

The ophthalmic imaging device may be configured to extract 3D information from the off-axis iris image and the further off-axis iris image using photogrammetry. The ophthalmic imaging device may be configured to use the extracted 3D information to determine the tilt of the eye.

In an embodiment, the sensor and the optical system of the off-axis imaging unit are arranged such that the iris lies substantially within a depth of field of the optical system.

The depth of field is a distance range where the off-axis iris image is in focus. Whether something is considered to be in focus or not may be defined using a cut-off defined by a level of angular or spatial resolution in the image, a level of sharpness, etc.

The cornea may be considered when arranging the optical system.

The optical system may have an imaging axis which imaging axis of the optical system has a predetermined tilt angle with respect to a surface normal of the image plane. The optical system may have two optical axes (an entry axis and an exit axis).

The optical system may have an imaging axis which imaging axis of the optical system is parallel to a surface normal of the sensor and wherein the optical system has a lateral shift, with respect to a center of the image plane, of a predetermined shift distance in a direction parallel to the image plane.

The sensor may be arranged such that the off-axis iris imaging unit is arranged facing the iris of the eye.

The surface normal of the sensor (image plane) of the off-axis iris imaging unit may be oriented substantially in parallel to the measurement axis of the on-axis measurement unit.

In addition to an ophthalmic imaging device, the present disclosure also relates to a method for imaging an eye using an ophthalmic imaging device. The method comprises recording, using an on-axis measuring unit of the ophthalmic imaging device, one or more measurements of the eye. The on-axis measuring unit is configured to measure the eye along a measurement axis substantially coincidental to an eye axis of the eye. The method comprises recording, using an off-axis iris imaging unit of the ophthalmic imaging device, an off-axis iris image of an iris of the eye. The off-axis iris imaging unit is arranged at a predetermined angle to the measurement axis. The off-axis iris imaging unit comprises a sensor defining an image plane. The off-axis iris imaging unit comprises an optical system oriented relative to the image plane such that a focal plane of the off-axis iris imaging unit substantially coincides with an iris plane of the iris of the eye.

In addition to the ophthalmic imaging device and the method for imaging an eye using an ophthalmic imaging device, the present disclosure also relates to a computer program product comprising program code configured to control a processor of an ophthalmic imaging device such that the ophthalmic imaging device performs a number of steps. The steps include recording, using an on-axis measuring unit of the ophthalmic imaging device, one or more measurements of the eye. The on-axis measuring unit is configured to measure the eye along a measurement axis substantially coincidental to an eye axis of the eye. The method comprises recording, using an off-axis iris imaging unit of the ophthalmic imaging device, an off-axis iris image of an iris of the eye. The off-axis iris imaging unit is arranged at a predetermined angle to the measurement axis. The off-axis iris imaging unit comprises a sensor defining an image plane. The off-axis iris imaging unit comprises an optical system oriented relative to the image plane such that a focal plane of the off-axis iris imaging unit substantially coincides with an iris plane of the iris of the eye.

The present disclosure also relates to a non-transitory memory comprising program code configured to control a processor of an ophthalmic imaging device such that the ophthalmic imaging device performs a number of steps. The steps include recording, using an on-axis measuring unit of the ophthalmic imaging device, one or more measurements of the eye. The on-axis measuring unit is configured to measure the eye along a measurement axis substantially coincidental to an eye axis of the eye. The method comprises recording, using an off-axis iris imaging unit of the ophthalmic imaging device, an off-axis iris image of an iris of the eye. The off-axis iris imaging unit is arranged at a predetermined angle to the measurement axis. The off-axis iris imaging unit comprises a sensor defining an image plane. The off-axis iris imaging unit comprises an optical system oriented relative to the image plane such that a focal plane of the off-axis iris imaging unit substantially coincides with an iris plane of the iris of the eye.

### BRIEF DESCRIPTION OF THE DRAWINGS

The herein described disclosure will be more fully understood from the detailed description given herein below and the accompanying drawings which should not be considered limiting to the invention described in the appended claims. The drawings in which:
- Fig. 1: shows a drawing of an ophthalmic imaging device;
- Fig. 2: shows a schematic optical diagram including an on-axis imaging unit, an off-axis imaging unit, and an eye;
- Fig. 3: shows a drawing of an on-axis view (also referred to as a top view or front-on view) of an eye;
- Fig. 4: shows a drawing of an off-axis view (also referred to as a side view) of an eye;
- Fig. 5: shows two drawings of the eye, the first drawing being of an off-axis view of the eye, and the second drawing being an on-axis view of the eye generated by transforming the off-axis view;
- Fig. 6: shows a schematic optical diagram according to an embodiment, in which the sensor plane, optical plane and iris plane are co-parallel;
- Fig. 7: shows a schematic optical diagram according to an embodiment, in which the sensor plane, optical plane and iris plane are angled with respect to each other and all meet at a defined point (respectively, a line perpendicular to the imaging axis);
- Fig. 8: shows a schematic optical diagram according to an embodiment, in which the sensor plane, optical plane, iris plane and a corneal plane are angled with respect to each other and all meet at a defined point (respectively, a line perpendicular to the imaging axis);
- Fig. 9: shows a block diagram illustrating schematically an ophthalmic imaging device including an on-axis measuring unit and an off-axis measuring unit;
- Fig. 10: shows a flow diagram illustrating a method for recording one or more measurements of an eye using an on-axis measuring unit, and recording an off-axis iris imaging using an on-axis imaging unit;
- Fig. 11: shows a flow diagram illustrating a method for correcting the off-axis iris image;
- Fig. 12: shows a flow diagram illustrating a method for generating an untilted image; and
- Fig. 13: shows a flow diagram illustrating a method for generating a single iris image using multiple off-axis iris images.

### DESCRIPTION OF THE EMBODIMENTS

Reference will now be made in detail to certain embodiments, examples of which are illustrated in the accompanying drawings, in which some, but not all features are shown. Indeed, embodiments disclosed herein may be embodied in many different forms and should not be construed as limited to the embodiments set forth herein; rather, these embodiments are provided so that this disclosure will satisfy applicable legal requirements. Whenever possible, like reference numbers will be used to refer to like components or parts.

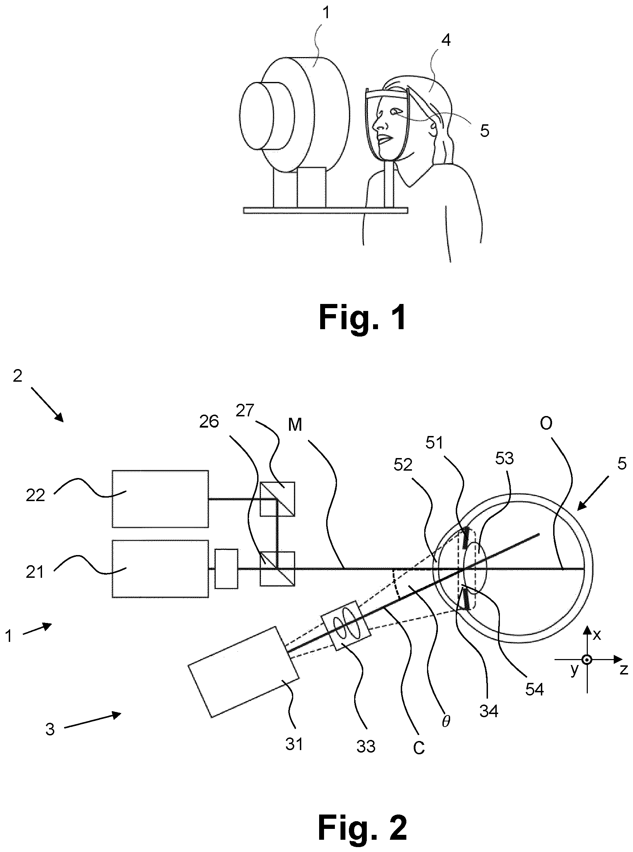

**Figure 1** shows a drawing of an ophthalmic imaging device 1. The ophthalmic imaging device 1 is an apparatus configured to measure characteristics of an eye 5 of a patient 4. The patient 4 may be sitting or standing in an upright position in front of the ophthalmic imaging device 1. The patient 4 may also be lying in a reclined position below the ophthalmic imaging device 1.

The ophthalmic imaging device 1 is preferably implemented in a self-contained desktop unit. The ophthalmic imaging device 1 may be connected to power for supplying power to the device 1, and may be connected a communication network, wirelessly and/or wired, for exchanging data across the communication.

The ophthalmic imaging device 1 may be installed and used at an ophthalmic facility by an ophthalmic professional, such as an ophthalmologist or an ophthalmic assistant. The ophthalmic imaging device 1 may be used for measuring and/or imaging the eye 5 of the patient 4 to provide information about the eye 5 such that a medical determination is possible, for example a determination as to whether treatment of the eye 5 may be necessary and if so, which type of treatment may be required and with which parameters. The ophthalmic imaging device 1 may therefore be used for determining whether treatment is necessary, for planning a treatment of the eye 5, immediately prior to treatment for any last minute checks or confirmations of the measured parameters, and/or for any follow-ups, for example for determining whether treatment was successful.

In an embodiment, the ophthalmic imaging device 1 is preferably configured exclusively for measuring, imaging, and/or otherwise recording characteristics of the eye 5.

**Figure 2** shows a schematic optical diagram including an on-axis imaging unit 2 and an off-axis imaging unit 3 of an ophthalmic imaging device 1, and an eye 5 shown in lateral cross-section.

The cross-sectional view of the eye 5 shows in particular a cornea 52, an iris 51, and a lens 53 of the eye 5. Additionally, an iris plane 54 is shown. The iris plane 54 extends in the x-y plane, intersecting the iris 51. The iris plane 54, by virtue of the iris 51 itself not being perfectly flat, may be defined using one or more positions along the z-axis and/or a single position along the z-axis, which single position includes a tolerance range, preferably designed to take into account the uneven nature of the surface of the iris 51.

The eye axis O of the eye 5 is substantially perpendicular to the cornea 52 and intersects the center of the entrance pupil. The eye axis O may correspond with the direction the eye 5 is looking, i.e. the line of sight. The eye axis may also be referred to as a visual axis of the eye. The eye axis O may also correspond substantially with an optical axis of the eye, or deviate from the optical axis of the eye by an angle, in particular a fixed angle.

The on-axis imaging unit 2 may comprise a plurality of measuring units 21, 22 which are configured to measure the eye 5 as viewed along a measurement axis M, the measurement axis M substantially coincident with the eye axis O of the eye. The on-axis imaging unit 2 may include beam splitters 26 and/or aperture dividers 26 which direct light received from the eye 5 along the measurement axis M to the individual measuring units 21, 22, each individual measurement unit 21, 22 receiving a portion of the light. The on-axis imaging unit 2 may further include one or more mirrors 27 to direct light to appropriate measuring units 21, 22.

The on-axis imaging unit 2 as depicted includes a first measurement unit 21 arranged behind a beam splitter 26. A second measurement unit 22 is arranged downstream of the beam-splitter 26 and downstream of the mirror 27.

Depending on the embodiment, the first measurement unit 21 and/or the second measurement unit 22 may be selected from a list of measuring units 21, 22 including the following: an optical coherence tomography unit, a placido imaging unit, an aberrometer, an optical biometer, and/or an auto-refractor. Although only two measuring units 21, 22 are depicted, further measuring units may of course be implemented.

The on-axis measurement unit 2 is configured to record one or more measurements of the eye 5. Depending on the implementation of the on-axis measurement unit 2, each of the measurement units 21, 22 may record one or more measurements.

For example, if one of the measurements units 21, 22 is an optical coherence tomography (OCT) unit, then the measurements may include one or more OCT A-Scans and/or one or more OCT-B scans. If one of the measurements units is an infrared placido imaging unit, the measurements may include a black-and-white, greyscale, and/or color image of the eye 5 used for determining characteristics of the cornea 52.

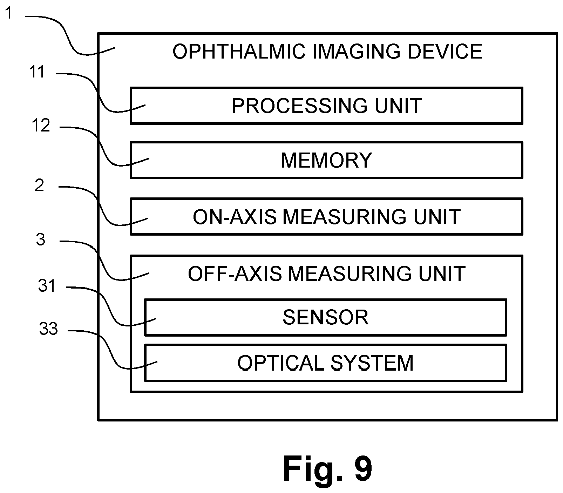

The on-axis measurement unit 2 is configured to provide the one or more recorded measurements to one or more further components of the ophthalmic imaging device 1, in particular the processing unit 11 described with reference to Figure 9 in more detail.

The on-axis measurement unit 2 may include one or more light sources configured to illuminate the eye 5. The light sources may be associated and/or controlled in conjunction with one or more particular measurement units 21, 22. For example, for placido imaging, concentric rings may be projected onto the eye 5 for measuring the curvature of the cornea 52. The one or more light sources may include a fixation light onto which the patient 4 fixes his gaze.

Alternatively or additionally, the light sources may be generic or universal, for example a ring light centered about the measurement axis M configured for even illumination of the eye 5.

The off-axis imaging unit 3 is arranged at an angle *θ* to the measurement axis M. In particular, the off-axis imaging unit 3 is arranged to image the iris 51 of the eye 52 along an imaging axis C, which imaging axis C forms an angle *θ* to the measurement axis M, wherein the imaging axis C and the measurement axis M intersect in the iris plane 54. The angle *θ* is preferably between 45° and 10°, more preferably between 35° and 20°.

The position of the off-axis imaging unit 3 may, alternatively or additionally, be defined using one or more distances, including a first distance along the x-direction between the off-axis measurement unit 3 and the measurement axis M, a second distance between the off-axis measurement unit 3 and the eye 5 along the z-direction (i.e. a direction parallel to the measurement axis M), and/or a distance between the off-axis measurement unit 3 and the eye 5.

The off-axis iris imaging unit 31 is preferably arranged such that the distance (i.e. the distance) to the eye 51 is between 3 cm and 10 cm, preferably approximately 5 cm, from the eye 51, in particular when the angle *θ* is 45°. The off-axis iris imaging unit 31 may alternatively be arranged, for example, such that the distance to the eye 51 is between 50 mm and 500 mm, in particular when the angle *θ* is 10°.

The off-axis iris imaging unit 3 is preferably arranged horizontally adjacent to the on-axis measurement unit 2, i.e. in the x-z plane. The off-axis iris imaging unit 3 may be arranged in the ophthalmic imaging device 1 such that it can be moved from a first side of the off-axis iris imaging unit 3 to the opposite side of the off-axis iris imaging unit 3. In particular, the off-axis iris imaging unit 3 may have a first position to the left of the on-axis measurement unit 2 when viewed from the perspective of the patient 4 (i.e. the first position is in the negative x-direction relative to the on-axis measurement unit 2), and a second position to the right of the on-axis measurement unit 2 when viewed from the perspective of the patient 4 (i.e. the second position is in the positive x-direction relative to the on-axis measurement unit 2).

In an embodiment, the ophthalmic imaging device 1 comprises a plurality of off-axis iris imaging units 3. For example, the ophthalmic imaging device 1 comprises two off-axis iris imaging units 3 arranged on opposite sides of the ophthalmic imaging device 1. A first off-axis iris imaging unit 3 is arranged on the left side of the ophthalmic imaging device 1 with an angle *θ* to the measurement axis M, and having a first azimuthal angle *ϕ*₁ about the measurement axis M (i.e. an angular position in the x-y plane about the measurement axis M). A second off-axis iris imaging unit 3 is arranged on the right side of the ophthalmic imaging device 1, at a second azimuthal angle *ϕ*₂ about the measurement axis M. The first off-axis imaging unit 3 is arranged at a first angle *θ*₁ to the measurement axis M, the second off-axis imaging unit 3 being arranged at a second angle *θ*₂ to the measurement axis M. In an embodiment, the first angle *θ*₁ and the second angle *θ*₂ are the same. Alternatively, the first angle *θ*₁ and the second angle *θ*₂ are different.

The off-axis imaging unit 3 includes a sensor 31. The sensor 31 defines an image plane 32 (not shown). The sensor 31 is configured to record one or more images. The sensor 31 is implemented, for example, as a CMOS sensor. The sensor 31 may alternatively be implemented as a CCD sensor. In an example, the sensor 31 may be arranged facing the eye 5, i.e. the surface normal of the image plan 32 may intersect the eye 5, in particular the iris plane 54 in a region of the iris 51. In another example, the sensor 31 may be arranged such that it does not face directly towards the eye 5. Specifically, the surface normal of the image plane 32 of the sensor 31 may intersect the eye axis O at a point behind the iris plane 54, i.e. further along the positive z-direction.

The off-axis imaging unit 3 includes an optical system 33 arranged in front of the sensor 31, i.e. between the sensor 31 and the eye 5. The optical system 33 may include one or more lenses, mirrors, and/or other optical elements. The optical system 33 is configured to focus the image of the iris 51 onto the sensor 31.

The optical system 33 may define and/or be oriented along the imaging axis C.

The optical system 33 is configured such that the focal plane 34 coincides with the iris plane 54. The focal plane 34 may be part of a focal region as depicted by the dotted line, all of which may be considered to be in focus. Thereby, the iris 51, which is by nature not perfectly flat (i.e. has a surface unevenness) is in focus and sharp. In particular, the optical system 33 (in particular, the focal length and the aperture) is configured such that the focal region has a focal depth (also referred to as a depth of field) along the z-direction of at least 0.5 mm, preferably a focal depth of at least 1 mm. The focal depth may be defined by a resolution (spatial and/or angular resolution), a level of local sharpness and/or contrast, etc.

The optical system 33 may be configured to resolve a particular object volume at a pre-defined resolution. The object volume is defined by the focal plane 34 and a focal depth in front, and behind, the focal plane 34. The object volume may further have one or more defined lateral edges, e.g., the object volume may have a cuboid or cylindrical shape. The focal plane 34 may be coplanar with the iris plane 54, however it may deviate from coplanarity, as long as the iris 51 is sufficiently resolved according to the pre-defined resolution.

The off-axis imaging unit 3, in particular the sensor 31 and/or optical system 33, may be configured such that the iris 51 is imaged with a defined spatial and/or angular resolution. For example, the sensor 31 may be configured to have a sufficiently dense arrangement of photodetectors, and the optical system 33 may be configured to have a sufficient angular resolving power such that a lateral resolution of between 5 and 50 micrometers, preferably greater than 15 micrometers per pixel is achieved. Preferably, the sensor 31 has a resolution of at least 1000 pixels by 1000 pixels.

The off-axis imaging unit 3 is configured to provide the one or more off-axis iris images to one or more other components of the ophthalmic imaging device 1, in particular the processing unit 11, as described below in more detail with reference to Figure 9.

In an embodiment, the sensor 51 and the optical system 33 are integrated into a single unit. In particular, the sensor 51 and the optical system 33 share a common housing.

The off-axis iris imaging unit 3 may further include one or more light sources for illuminating the eye 5, in particular for illuminating the iris 51. The light sources may include point light sources and/or light sources configured for broad and even illumination. The light sources may be configured to project particular patterns onto the eye 5, including, for example lines and/or curves.

Depending on the implementation, the optical system 33 may include one or more optical axes, for example an entry axis and an exit axis, which may not be coincidental.

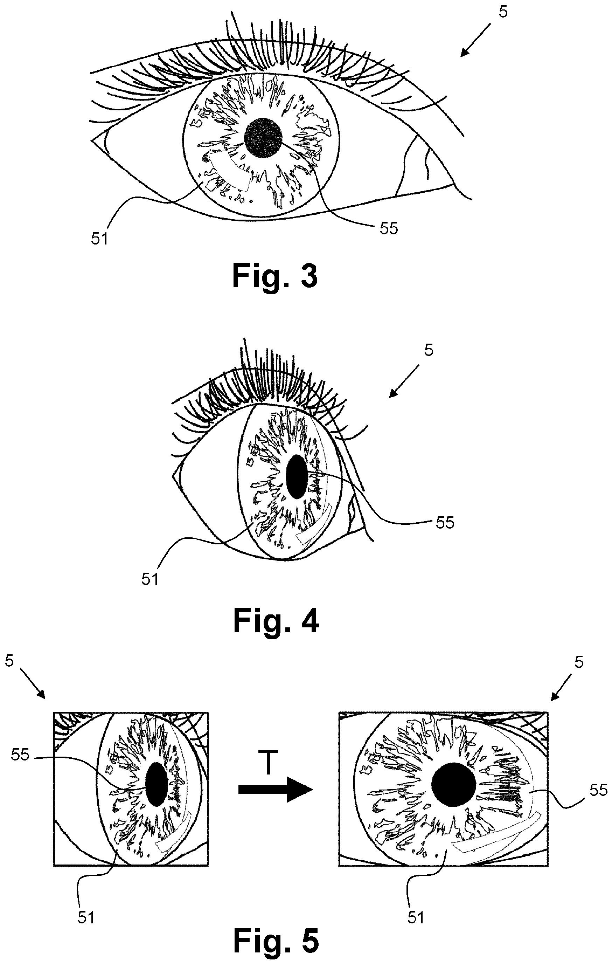

**Figure 3** shows a drawing of an on-axis view of the eye 5 of the patient. The on-axis view may also be referred to as a top view or front-on view of the eye 5. The iris 51 as well as the pupil 55 is visible.

**Figure 4** shows, on the left, a drawing of an off-axis iris image of the eye 5 of the patient. The off-axis iris image, i.e. an image of the eye 5 when viewed off the eye axis O of the eye, may also be referred to as a side view of the eye 5. The off-axis iris image is distorted due to the perspective, in particular because the viewing angle of the eye 5 is not front on, but angled by an angle, for example the angle *θ* of the off-axis imaging unit 3 to the measurement axis M (or equivalently to the eye axis O of the eye 5).

**Figure 5** shows, in the left drawing of the eye 5, an off-axis iris image of the eye 5 as recorded, for example, by an off-axis imaging unit 3 arranged to view the eye from the perspective depicted in Figure 4. The off-axis iris image may be corrected, for example by using a transformation T, to generate a corrected off-axis iris image of the eye 5 (shown on the right hand side). The corrected off-axis iris image, while being an improvement over the left (initial) off-axis iris image, is not entirely equivalent to an on-axis iris image recorded by a camera arranged on the eye axis O of the eye 5. In particular, the far side of the eye, i.e. in this case the right hand side of the eye, is not perfectly reconstructed in the correct off-axis iris image. This is due to the features on the right hand side of the image being recorded in less detail, in compressed form, and/or being at least partially occluded in the off-axis iris image. The example of the off-axis iris image shown in the left of Figure 4, however, may be taken as an extreme example and the result of recording the off-axis iris image at a large angle *θ* from the eye axis O. The smaller the angle *θ*, the less detrimental the loss of information and the better the corrected off-axis iris image. The corrected off-axis iris image may also be referred to as a reconstructed on-axis iris image.

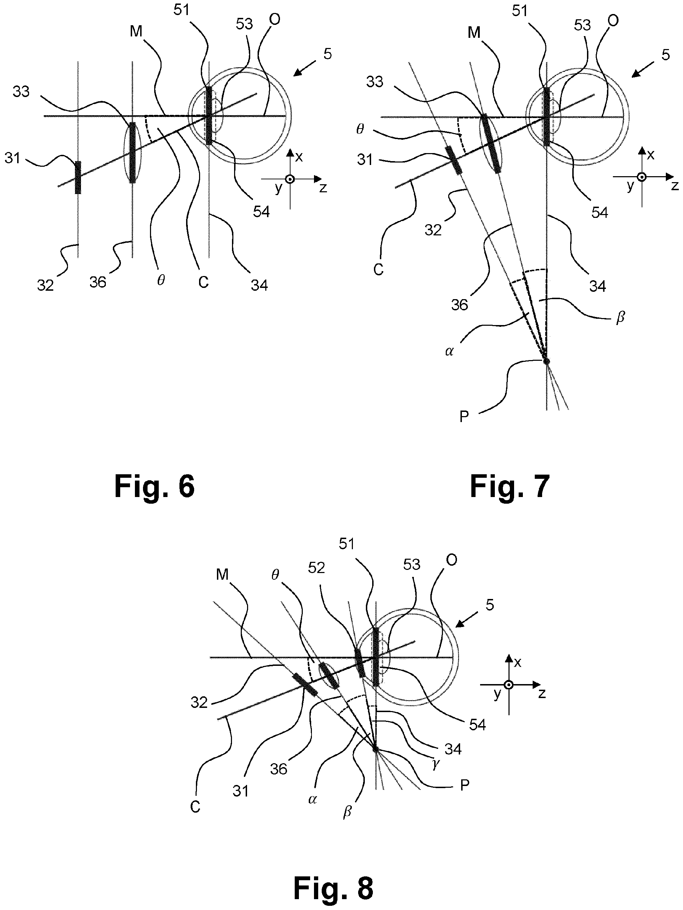

**Figures 6** to **8** show optical diagrams, schematically illustrating features of the off-axis imaging unit 3 and the eye 5 according to embodiments of the present disclosure. For improved imaging of the eye 5 when viewed off-axis, i.e. when viewed at an angle *θ* to the eye axis O of the eye 5, the optical system 33 of the off-axis iris imaging unit 3 is oriented relative to the image plane 32 of the sensor 31 such that a focal plane 34 of the off-axis iris imaging unit 3 substantially coincides with the iris plane 54 of the iris 51 of the eye 5.

In particular, a sensor normal, i.e. a vector perpendicular to the image plane 32 of the sensor 31 and passing through a center of the sensor 31, does not coincide with the imaging axis C of the optical system 33. This results in a shift and/or a tilt of the obtained off-axis iris image, thereby at least partially compensating for a distortion present in the off-axis iris image due to perspective.

The arrangement of the optical system 33 and the sensor 31 is designed according to the Scheimpflug principle such that the focal plane 34 substantially coincides with the iris plane 54. Multiple such arrangements are possible, some of which are explained in detail with reference to **Figures 6** to **8** below. The skilled person understands that, based on the principles and examples explained and shown herein, other arrangements are possible.

In **Figure 6****,** the optical system 33, laterally shifted with respect to the sensor 31. In particular, the optical system 33 is shifted laterally by a distance of 0.2 mm to 20 mm towards the measurement axis M, i.e. in the positive x-direction. Thereby, the focal plane 34 of the optical system 33 substantially coincides with the iris plane 54 and the iris 51 is in sharp focus on the image plane 32 of the sensor 31. The image plane 32 of the sensor 31 and a main optical plane 36 of the optical system 33 are arranged in parallel to each other, and in parallel to the iris plane 54. The imaging axis C, which is at an angle *θ* with respect to the measurement axis M, intersects the center of the sensor 31, a center of the optical system 33, and a center of the iris 51.

In **Figure 7****,** the optical system 33 is angled with respect to the sensor 31 by an angle *α*. In particular, a main optical plane 36 of the optical system 33 forms the angle *α* with respect to the image plan 32 of the sensor 31. The main optical plane 36 further forms an angle *β* with respect to the focal plane 34. The image plane 32, main optical plane 36 and focal plane 34 meet at point P in the x-z plane. The angles *α*, *β* are selected such that the focal plane 34 substantially coincides with the iris plane 54.

The main optical plane 36 may be a main plane of an optical system, in particular the optical system 33.

It is understood that the optical system 33 may have more than one optical plane, i.e. that it may be possible to define, in addition to the main optical plane 36, one or more further optical planes of the optical system 33, depending on the particular configuration of the optical system 33. Alternatively or additionally, it may be possible to define the orientation of the optical system 33 by one or more optical axes (not shown) of the optical system 33, thereby defining one or more angles between the optical axes of the optical system 33 and an image plane normal, i.e. a vector perpendicular to, and passing through a center of, the image plane 31. The angle *α* may be formed between one of the optical axes of the optical system 33 and the image plane normal. Additionally, the angle *β* may be formed between one of the optical axes of the optical system 33 and the eye axis O.

In **Figure 8****,** the optical design of the off-axis imaging unit 3 closely mirrors the optical design shown in Figure 7, however the optical characteristics of the cornea 52 are accounted for. In particular, the optical system 33 is designed to take into account at least the refractive properties of a default cornea 52, for example as defined by a digital eye model. Thereby, distortion in off-axis iris image due to refractive properties of the cornea 52 is at least partly accounted for.

**Figure 9** shows a block diagram illustrating schematically an ophthalmic imaging device 1 including an on-axis measuring unit 2 and an off-axis measuring unit 3. The ophthalmic imaging device 1 comprises a processing unit 11 and a memory 12. The processing unit 11 is connected to the on-axis measuring unit 2, the off-axis measuring unit 3, and the memory 12. The processing unit 11 is configured to receive, retrieve, and/or otherwise obtain, from the on-axis measuring unit 2, the one or more measurements of the eye 5 recorded by the on-axis measuring unit 2. The processing unit 11 is configured to receive, retrieve, and/or otherwise obtain, from the off-axis imaging unit 3, the off-axis iris image of the iris 51 of the eye.

The processing unit 11 comprises one or more central processing units (CPUs) and/or other programmable circuits or logic units such as ASICs (Application-Specific Integrated Circuits), for example FPGAs (field programmable array), GPUs (graphics processing units) and TPUs (tensor processing units). The memory 12 comprises volatile and/or non-volatile memory, e.g., random-access memory and/or flash memory having stored thereon program code, data, as well as programmed software modules for controlling the processing unit 11 as described herein. In particular, the memory 12 may store software applications, libraries, algorithms or other routines for processing the one or more measurements of the eye 5 and/or the off-axis iris image, in particular according to the steps and/or functions described herein.

Additionally, the memory 12 is configured to store patient data, in particular the one or more recorded measurements of the eye 5 and the off-axis iris image.

The memory 12 may further comprise one or more digital eye models. The digital eye model is a digital representation of an eye. The digital eye model may include a 2 dimensional and/or 3 dimensional model of an eye, i.e. include 2D and/or 3D information related to an eye.

The digital eye models may include a parametrized digital eye model which includes parameters for characteristics of the eye. The characteristics of the eye include a diameter of the eye bulbus, a curvature of an anterior corneal surface, a curvature of a posterior corneal surface, a thickness profile of a cornea, an astigmatism of the cornea, and/or a curvature of the iris. The digital eye models may include a personalized digital model. The personalized digital eye model may be generated using the one or more measurements of the eye and/or the one or more off-axis iris images of the eye.

The ophthalmic imaging device 1 may include a communication interface configured for data communication with one or more external devices. Preferably, the communication interface comprises a network communications interface, for example an Ethernet interface, a WLAN interface, and/or a wireless radio network interface for wireless and/or wired data communication using one or more networks, comprising, for example, a local network such as a LAN (local area network), and/or the Internet.

The skilled person is aware that at least some of the steps and/or functions described herein as being performed on the processor 11 of the ophthalmic imaging device 1 may be performed on one or more auxiliary processing devices connected to the processing unit 11 of the ophthalmic imaging device 1 using the communication interface. The auxiliary processing devices can be co-located with the ophthalmic imaging device 1 or located remotely, for example on an external device, such as a remote server computer (e.g., a cloud-based server).

The skilled person is also aware that at least some of the data associated with the program code (application data) or data associated with a particular person (patient data) and described as being stored in the memory 12 of the ophthalmic imaging device 1 may be stored on one or more auxiliary storage devices connected to the ophthalmic imaging device 1 using the communication interface.

The ophthalmic imaging device 1 optionally includes a user interface comprising, for example, one or more user input devices, such as a keyboard, and one or more output devices, such as a display. The user interface is configured to receive user inputs from an ophthalmic professional, in particular based on, or in response to, information displayed to the ophthalmic professional using the one or more output devices.

Depending on the embodiment, the ophthalmic imaging device 1 may include a personal computer, for example a desktop computer, a laptop computer, a tablet computer, or a smart phone.

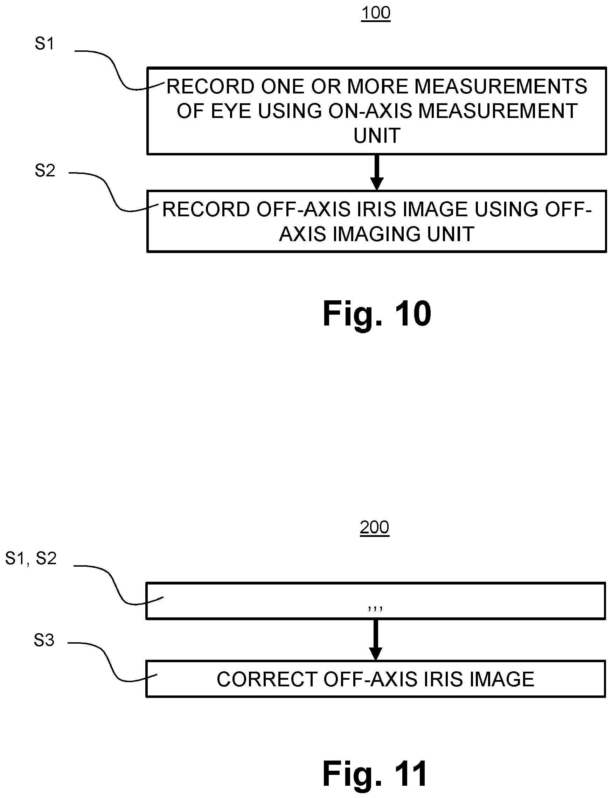

**Figure 10** shows a method 100 for recording one or more measurements of an eye using an on-axis measuring unit, and recording an off-axis iris imaging using an off-axis imaging unit 3. The method 100 includes steps S1 and S2. The method 100 may be performed, by the ophthalmic imaging device 1, on demand by an ophthalmic professional, in particular with a patient 4 positioned in front of the ophthalmic imaging device 1. The method 100 may be performed to gather information about the eye 5 for assessment by the ophthalmic professional, in particular for subsequent planning of treatment of the eye 5. The method 100 may be performed post treatment to determine an outcome of any treatment performed. Typically, the method 100 is performed once for each eye 5 of the patient 4. The patient 4 may have to reposition themselves in front of the ophthalmic imaging device 1 according to the eye 5 being assessed.

In a step S1, one or more measurements of the eye are performed using the on-axis measurement unit 2. The measurements are recorded and transmitted, or otherwise provided, by the on-axis measurement unit 2, to the processing unit 11. A time-point of recording of the measurement may be included with the one or more measurements.

In a step S2, which may be performed prior, subsequent, or simultaneous to step S1, the off-axis iris imaging unit 3 records an off-axis iris image of the iris 51 of the eye 5. The off-axis iris image is recorded and transmitted, or otherwise provided, by the off-axis iris imaging unit 3, to the processing unit 11. A time-point of recording the off-axis iris image may be included with the off-axis iris image.

In particular, the ophthalmic imaging device 1 may be configured to record at least one of the measurements of the eye 5 and the off-axis iris image simultaneously, i.e. at the same time-point.

The processing unit 11 may store the received measurements and the off-axis iris image in the memory 21. The processing unit 11 may provide (e.g., by transmitting) the measurements and the off-axis iris image to an external device, using the communication interface. The processing unit 11 may display the measurements and the off-axis iris image on the display for assessment by the ophthalmic professional.

**Figure 11** shows a method 200 for correcting the off-axis iris image obtained during step S2 of the method 100 described above. The method 200 is performed by the ophthalmic imaging device 1, in particular using the processing unit 11, and may include steps S1, S2 as described above. The method 200 includes a step S3, which may be performed immediately subsequent to step S2, or may be performed at a further defined time point.

In step S2, the off-axis iris image is corrected, by transforming the image to at least partially compensate for any distortion present in the off-axis iris image. In particular, the distortion may relate to perspective, i.e. geometric distortion due to the iris having been imaged at an angle relative to the eye axis of the eye. To correct for perspective, the transformation may include an affine transformation.

The distortion may further relate to characteristics of the optical system 33 and/or characteristics of the eye.

The characteristics of the optical system include distortion (e.g., barrel distortion, pincushion distortion, trapeze distortion, etc.) and radial distortion that depends on wavelength (also known as chromatic aberration).

The characteristics of the eye include a curvature of the iris. The characteristics include optical characteristics, in particular refractive properties of the cornea (e.g., due to curvature of exterior corneal surface, material of cornea, thickness profile of cornea, curvature of interior corneal surface, and/or asphericity of cornea).

The transformation may use a geometric transformation and/or a projection designed to correct for distortions in the off-axis image.

The corrected off-axis iris image is stored in the memory 12.

In an embodiment, a digital eye model is used to correct for distortions, in particular for refractive properties of the cornea. The digital eye model may therefore include predefined default values enabling a correction for refracting properties of the cornea, in particular default values which reflect and/or depend on the angle *θ* between the off-axis imaging unit 3 and the measurement axis M. For example, digital eye model may be define parameters used for the transformation.

In another example, the digital eye model is configured to model how light travels through the eye, in particular through the cornea. The transformation may include ray tracing through the digital eye model to transform the off-axis iris image. In particular, the off-axis iris imaging may be corrected by taking into account how light reflected off the iris 51 and incident on the sensor 31 is refracted by the cornea.

The digital eye model may be a parametrized digital model taking into account at least one characteristic of the eye. The characteristics include a diameter of the eye 5, a curvature of an anterior corneal surface, a curvature of a posterior corneal surface, a thickness profile of the cornea 52, an astigmatism of the cornea 52, and/or a curvature of the iris 51.

In an embodiment, at least one of the characteristics of the eye is determined using the one or more measurements of the eye 5. For example, at least one of the characteristics of the eye may be determined from topography and/or tomography data provided in the one or more measurements. In particular, if the one or more measurement units 21, 22 includes an OCT unit, the tomography data recorded by the OCT unit 1 may be used to determine the curvature of the anterior corneal surface, the curvature of the posterior corneal surface, the thickness profile of the cornea 52, and/or the astigmatism of the cornea 52. Further, if the one or more measurement units 21, 22 includes a placido imaging unit, then the measurements may be used to determine the astigmatism of the cornea 52.

In an embodiment, the digital eye model may be personalized, using the one or more measurements of the eye and/or the off-axis iris image, to generate a personalized digital eye model.

The personalized digital eye model may include 2D and/or 3D information relating to one or more structures of the eye, in particular the cornea 52 and/or the iris 51. For example, the personalized digital eye model may include a corneal topography map of the cornea of the eye. The corneal topography map is a topography of the cornea, which may be augmented using the off-axis iris image. Specifically, the topography of the cornea may include an overlaid image of the iris based on the off-axis iris image. The corneal topography map may be converted into a 3D model of the cornea. The personalized digital eye model may include tomography data, for example as included in the measurements of the eye 5.

The personalized digital eye model may be displayed, in whole or in part, on the display. Thereby, the ophthalmic professional may assess the eye 5.

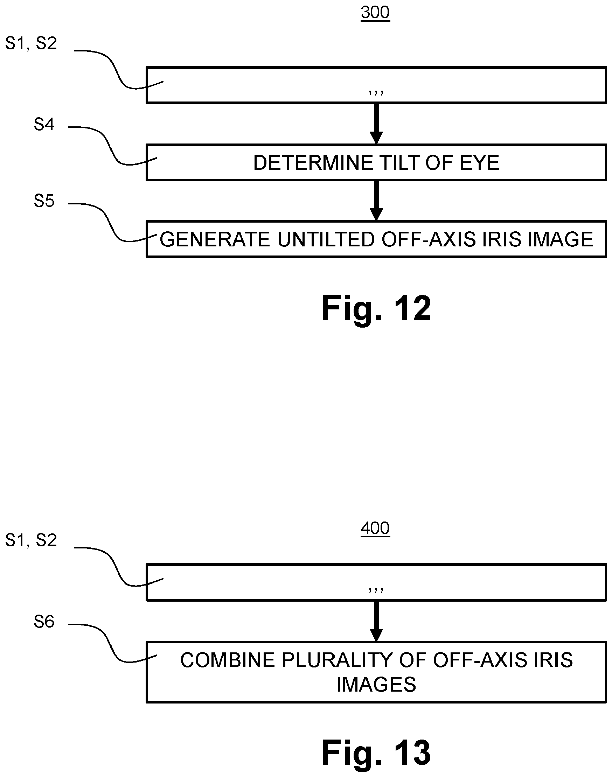

**Figure 12** shows a flow diagram illustrating a method 300 for determining the tilt of the eye 5. Although the patient 4 may be instructed to direct their gaze directly along the measurement axis M, the patient 4 may inadvertently look a little to the side, and/or natural and involuntary eye movements may cause the eye 5 to tilt. Therefore, the method 300 is useful for correcting the measurements and the off-axis image recorded in steps S1 and S2. The method 300 is performed by the ophthalmic imaging device 1, in particular the processing unit 11. The method 300 is shown to include steps S1 and S2 as described above with reference to method 100. The method 300 may further include steps of other methods described herein. The method 300 in particular includes steps S4 and S5.

In step S4, a tilt of the eye 5 is determined. The tilt of the eye 5 may be defined as an angle between the eye axis O of the eye 5 and the measurement axis M, the point of origin being the eye 5. The tilt of the eye 5 is determined using the off-axis iris image.

The tilt may be determined by locating, in the off-axis iris image, a center of the pupil 55 and comparing the position of the center of the pupil 55 to a center of the pupil 55 in an on-axis image and/or an on-axis measurement. The on-axis image may be generated by an on-axis measurement unit.

The location of the pupil may be compared to one or more reflections of light sources, in particular point-light sources, present in the off-axis iris image. A relative position of the one or more reflections with respect to the center of the pupil 55 may be used to determine the tilt. The location of the pupil may be compared to one or more reflections of structured light present in the off-axis iris image, the structured light generated by light sources configured to project a pattern onto the eye 5. The tilt may be determined, for example, using principles of triangulation.

The tilt of the eye may be determined using the one or more measurements and the off-axis iris image, in particular by identifying and locating one or more common features recorded in both the measurement(s) and the off-axis iris image. Determining the features may include taking into account light refraction through the cornea. For example, the features may include scleral structures such as veins, in particular adjacent and/or branching veins.

In step S5, an untilted off-axis iris image is generated by transforming the off-axis iris image based on the determined tilt of the eye.

In an embodiment, the ophthalmic imaging device 1 may include an eye tracker configured to measure the tilt of the eye.

**Figure 13** shows a method 400 for generating an iris image using multiple off-axis iris images. The method 400 is performed by the ophthalmic imaging device 1, in particular the processing unit 11. The method 400 includes a step S6, however, the method 400 includes at least steps S1, S2 of method 100 and may include one or more steps of other methods described herein. The method 400 is in particular performed in an embodiment in which the ophthalmic imaging device 1 includes two or more off-axis iris imaging units 3, preferably configured to each record an off-axis iris image simultaneously. For example, an off-axis iris imaging units 3 may be included on each side of the off-axis iris imaging unit 3 such that the eye 5, in particular the iris 51, is imaged from both sides. Thereby, for example, a shadowing of part of the iris 51 due to the limbus, may be compensated for.

In step S6, the two or more off-axis iris images are combined to generate a single iris image. For example, the single iris image is generated by first correcting each off-axis iris image, in particular correcting for distortion due to the perspective, to generate a pseudo top-view iris image. The single iris image may then be generated by identifying one or more common features of the iris 51 in each of the pseudo top-view iris images. The pseudo top-view iris images may then be transformed, e.g. including rotation and/or skewing such that the top-view iris images (in particular the identified common features) align, and then generating the single iris image by overlaying and/or otherwise merging or combing both pseudo top-view iris images. Preferably,

In an embodiment, 3D information is extracted, from the plurality of off-axis iris images, for example using photogrammetry techniques. The plurality of off-axis iris images may be recorded by a plurality of two or more off-axis iris imaging units 3, respectively. The 3D information may be stored in conjunction with the off-axis iris images or images or data derived and/or generated therefrom.

The above-described embodiments of the disclosure are exemplary and the person skilled in the art knows that at least some of the components and/or steps described in the embodiments above may be rearranged, omitted, or introduced into other embodiments without deviating from the scope of the present disclosure.

## Claims

1. Ophthalmic imaging device (1) comprising:
an on-axis measuring unit (2) configured to record one or more measurements of an eye (5), the on-axis measuring unit (2) configured to measure the eye (5) along a measurement axis (M) substantially coincidental to an eye axis (O) of the eye (5); and
an off-axis iris imaging unit (3) configured to record an off-axis iris image of an iris (51) of the eye (5), the off-axis iris imaging unit (3) arranged at a predetermined angle (*θ*) to the measurement axis (M) and comprising:
a sensor (31) defining an image plane (32), and
an optical system (33) oriented relative to the image plane (32) such that a focal plane (34) of the off-axis iris imaging unit (3) substantially coincides with an iris plane (54) of the iris (51) of the eye (5).

2. The ophthalmic imaging device (1) according to claim 1, further comprising a processing unit (11) configured to:
correct (S3) the off-axis iris image, by transforming the off-axis iris image to compensate for distortion caused by at least one of: perspective, characteristics of the optical system (33) or characteristics of the eye (5).

3. The ophthalmic imaging device (1) according to claim 2, wherein the processing unit (11) is configured to use a digital eye model to correct for refractive properties of the cornea (52).

4. The ophthalmic imaging device (1) according to claim 3, wherein the digital eye model is a parametrized digital eye model including at least one of the following characteristics of the eye (5): a diameter of the eye (5), a curvature of an anterior corneal surface, a curvature of a posterior corneal surface, a thickness profile of a cornea, an astigmatism of the cornea, or a curvature of the iris (51).

5. The ophthalmic imaging device (1) according to one of claims 2 to 4, wherein the processing unit (11) is configured to determine at least one of the characteristics of the eye (5) using the one or more measurements performed by the on-axis measuring unit (2).

6. The ophthalmic imaging device (1) according to one of claims 1 to 5, further comprising a processing unit (11) configured to:
determine (S4) a tilt of the eye (5), relative to the measurement axis (M), using the off-axis iris image; and
generate (S5) an untilted off-axis iris image by transforming the off-axis iris image based on the tilt of the eye (5).

7. The ophthalmic imaging device (1) according to one of claims 1 to 6, configured to simultaneously record at least one of the measurements of the eye (5) and the off-axis iris image.

8. The ophthalmic imaging device (1) according to one of claims 1 to 6, comprising a processing unit (11) configured to generate a personalized digital eye model of the eye (5) of the patient (4), using the one or more measurements of the eye (5) and the off-axis iris image.

9. The ophthalmic imaging device (1) of one of claims 1 to 8, wherein the on-axis measuring unit (2) comprises at least one of: an optical coherence tomography unit (21), an infrared placido imaging unit (22), an aberrometer (23), an optical biometer (24), or an auto-refractor (25).

10. The ophthalmic imaging device (1) of one of claims 1 to 9, further comprising a further off-axis imaging unit (3, 3B) arranged at a predetermined angle (*θ*₂) to the measurement axis (M) and at a predetermined azimuthal angle (*ϕ*₂) about the measurement axis (M) with respect to the azimuthal angle (*ϕ*₁) of the off-axis imaging unit (3, 3A), the further off-axis imaging unit (3, 3B) configured to record a further off-axis iris image of the iris (51) of the eye (5), and a processing unit (11) configured to merge the off-axis iris image and the further off-axis iris image to generate a combined off-axis iris image.

11. The ophthalmic imaging device (1) of one of claims 1 to 10, wherein the sensor (31) and the optical system (33) of the off-axis imaging unit (3) are arranged such that the iris (51) lies substantially within a depth of field of the optical system (33).

12. A method for imaging an eye (5) using an ophthalmic imaging device (1), the method comprising:
recording (S1), using an on-axis measuring unit (2) of the ophthalmic imaging device (1), one or more measurements of the eye (5), the on-axis measuring unit (2) configured to measure the eye (5) along a measurement axis (M) substantially coincidental to an eye axis (O) of the eye (5);
recording (S2), using an off-axis iris imaging unit (3) of the ophthalmic imaging device (1), an off-axis iris image of an iris (51) of the eye (5), the off-axis iris imaging unit (3) arranged at a predetermined angle (*θ*) to the measurement axis (M) and comprising:
a sensor (31) defining an image plane (32), and
an optical system (33) oriented relative to the image plane (32) such that a focal plane (34) of the off-axis iris imaging unit (3) substantially coincides with an iris plane (54) of the iris (51) of the eye (5).

13. A computer program product comprising program code configured to control a processor of an ophthalmic imaging device such that the ophthalmic imaging device performs the following steps:
recording (S1), using an on-axis measuring unit (2) of the ophthalmic imaging device (1), one or more measurements of the eye (5), the on-axis measuring unit (2) configured to measure the eye (5) along a measurement axis (M) substantially coincidental to an eye axis (O) of the eye (5);
recording (S2), using an off-axis iris imaging unit (3) of the ophthalmic imaging device (1), an off-axis iris image of an iris (51) of the eye (5), the off-axis iris imaging unit (3) arranged at a predetermined angle (*θ*) to the measurement axis (M) and comprising:
a sensor (31) defining an image plane (32), and
an optical system (33) oriented relative to the image plane (32) such that a focal plane (34) of the off-axis iris imaging unit (3) substantially coincides with an iris plane (54) of the iris (51) of the eye (5).
